# EUROPEAN PATENT APPLICATION

(11) **EP 4 382 098 A1**
(43) Date of publication of application: **12.06.2024**
(21) Application number: 22852468.2
(22) Date of filing: 05.08.2022
(51) Int. Cl.: A61K 31/12, A61K 31/575, A61K 36/9066, A61K 9/107, A61K 47/10, A61K 47/14, A61K 47/24, A61K 47/69, A61P 27/02, A61P 27/12

(54) **OPHTHALMIC COMPOSITION FOR THE TREATMENT OF VISUAL DISORDERS**

(30) Priority: 06.08.2021 MX 2021009520
(71) Applicant: Foodvica, S.A. De C.V., Boca del Río, Veracruz, 94294 (MX)
(72) Inventor: CANO-SARMIENTO, Cynthia, Veracruz Veracruz, 91940 (MX); CASTILLO-OLMOS, Ana Guadalupe, Veracruz Veracruz, 91757 (MX); GARCÍA-GALINDO, Hugo Sergio, Veracruz Veracruz, 91919 (MX); MORALES-MENDOZA, Mizraim, Veracruz Veracruz, 91940 (MX)
(74) Representative: Novagraaf International SA
(86) International application number: PCT/IB2022/057330
(87) International publication number: WO 2023/012754

(57) **Abstract**

This invention provides an ophthalmic composition for the treatment and/or prevention of visual disorders caused by changes in the eye's lens structure, particularly related to the alteration of the proteins that form the lens. The ophthalmic composition consists of a polyphenolic compound in combination with a terpenoid, or its salts or crystalline forms, in therapeutically effective amounts, requiring low doses of bioactive compounds to treat the disease through a reduced number of applications. Specifically, the ophthalmic composition of the invention may be employed for the treatment and reversal of visual disorders caused by alterations in the structure of the crystalline lens, such as cataracts, by being able to reverse the opacity of the lens caused by the disease.

## Description

### FIELD OF INVENTION

This invention relates to health care, and more particularly to ophthalmic compositions for the treatment of visual disorders, especially those affecting the regular function of the eye's lens.

### BACKGROUND OF THE INVENTION

Visual disorders affecting the regular structure of the eye's lens are those conditions that modify or alter the proteins making up the lens and cause visual impairment. Changes are often made in the clarity and/or stiffness of the lens, which is caused by aggregation of lens proteins (crystallins). A recurrent condition of this kind is known as "cataracts." The term "cataract" referred to in this invention means a disease causing an opacity in the eye's lens decreasing the amount of light entering, thus causing visual impairment, resulting from changes in the structure and/or aggregation of the proteins present. Cataract formation may be congenital, juvenile, age-related or secondary to diseases (diabetes, myotonic dystrophy, galactosemia, neurofibromatosis type 2, rubella, among others), trauma, etc. Cataracts may be nuclear, cortical and posterior subcapsular. However, there are other diseases related to alterations in the lens structure, such as presbyopia, lens sclerosis, retinal degeneration, Refsum disease, Smith-Lemli-Opitz syndrome (SLOS), drusen, Schnyder corneal dystrophy (SCD), abetalipoproteinemia (ABL), familial hypobetalipoproteinemia (FHBL), age, macular degeneration and diabetic retinopathy.

The treatment of these conditions depends on the original condition, so all pathologies that may predetermine the formation of these conditions should be resolved first. The symptoms of some of these conditions, such as an initial cataract, may be reduced by the use of prescription lenses, anti-reflective lenses for the sun, or the use of drugs such as drops, ointments and pills.

Another treatment alternative is surgery. It should be noted that for surgery to be performed, the cataract should be at a maturity or opacity stage, before which corrective measures through the use of lenses are only intended to reduce the refractive errors resulting from the opacity developing. It is worth mentioning that cataract surgery, which consists of removing the lens and replacing it with an artificial intraocular lens, shows disadvantages such as high costs and side effects, such as posterior capsule rupture and corneal edema.

Due to the issues caused by surgery, the state of the art has explored the possibility of using bioactive compounds in the treatment of visual disorders.

Perhaps the most studied bioactive compounds to date have been terpenoids, particularly lanosterol, whose use in cataract reversal has not been conclusively demonstrated. Regarding this compound, the most successful experiment was performed by Zhao *et al.* (Zhao et al. Lanosterol Reverses Protein Aggregation in Cataracts. Nature. 2015; 523: 607-611). They were able to demonstrate that the use of 14 intravitreal injections loaded with lanosterol nanoparticles (with a final dose of 1.4 mg), while providing 50 µL of lanosterol drops 3 times a day for 6 weeks to a group of 7 dogs, was able to reduce up to two degrees the stage of cataracts these animals had. Unfortunately, the number of injections required to get the results is too large, so it does not significantly reduce the risks arising from surgery nor does it remove the issue in advanced cases.

Following the efforts of Zhao *et al.,* other authors have not come close to getting similar results. Chen *et al.* (Chen et al. Lanosterol and 25-hydroxycholesterol Dissociate Isolated Crystalline Aggregates from the Human Cataract Lens Through Different Mechanisms. Biochemical and Biophysical Research Communications. 2018; 506 (4):868-873) confirmed in their studies that lanosterol may have positive *in vitro* activity, 2 mg/mL of protein aggregates obtained from surgically removed cataracts, to which 10 µM and 200 µM of lanosterol, as well as 10 µM and 200 µM of 25-hydroxycholesterol, were added for 14 days. Macroscopic remarks suggested that lanosterol and 25-hydroxycholesterol dramatically cleared the originally dark solutions in a dose-dependent way after a 6-day treatment at room temperature. The authors found that the effectiveness of these compounds is highly dependent on the degree or severity of the cataracts. However, the study suggested that, in the cortical cataract group, the values of the concentration needed to reach 50% of the estimated average maximum generated effect (EC50) increased 1.5 to 4 times compared with the nuclear opalescence group. In addition, the amount of proteins released decreased along with the increase in severity. This study shows limitations and confirms the requirement for high dose and frequency of treatment, as reported by Zhao *et al.* It is also likely that the compounds may not penetrate the interior of the lens with severe nuclear cataracts. Therefore, in practice, the effective concentrations of the compounds for human lens with mature or dense cataracts could be several hundred times higher than the EC50 values determined ex *vivo.*

Daszynski *et al.* (Daszynski et al. Failure of Oxysterols such as Lanosterol to Restore the Clarity of the Cataract Lens. Scientific Reports. 2019; 9:8459) performed *in vitro* cataract reversal studies on rat lens, along with protein solubilization studies on human lens only. 15 mM lanosterol in liposomes failed to reverse the opacities or prevent progression. By contrast, the opacities of all lanosterol-treated lens progressed to a more advanced mature cataract stage with apparent nuclear involvement. Nagai *et al.* (Nagai et al. Intravitreal Injection of Lanosterol Nanoparticles Rescues Collapse of Lens Structure at an Early Stage in Shumiya Cataract Rats. International Journal of Molecular Sciences. 2020; 21 (3):1048) developed an intravitreal injection formulation based on the findings of Zhao *et al.* containing lanosterol nanoparticles (LAN-NPs) by using a ball-milling approach. They assessed the therapeutic effect of LAN-NPs on lens structure collapse and opacification by using two rat cataract models (SCR-N, rats with slight lens structure collapse) (SCR-C, with combination of remarkable lens structure collapse and opacification). Unfortunately, LAN-NPs did not repair the lens structure; they only delayed the opacification onset. It was not possible to ameliorate lens severe structural collapse with a lanosterol supplement using LAN-NPs.

Similar results were also found by Shanmugam *et al.* (Shanmugam et al. Effect of Lanosterol on Human Cataract Nucleus. Indian Journal of Ophthalmology. 2015; 63 (12): 888-90). They studied the effect of lanosterol on age-related cataract human lens nuclei. They used 40 age-related cataract nuclei removed by manual small incision cataract surgery and randomly immersed 20 nuclei in 25 mM lanosterol solution, while the rest was stored in the dark under control solution without lanosterol for 6 days. The conclusion was that 25 mM lanosterol solution did not reverse the opacification of age-related human cataract nuclei.

Patent US10471076B2 describes a method for treating or preventing a visual disorder by using lanosterol. The paper proposes a dose of the compounds to be administrated on a human being of about 70 kg body weight, by systemic route, from 0.1 mg to 5 g; e.g., from 1 mg to 2.5 g of the compound per unit dose. However, in order to assess lanosterol effect on cataract reduction in lens tissues, natural cataract lens of rabbits were isolated and incubated in a solution with a concentration of 25 mM lanosterol for 6 days. Subsequently, lens clarity before and after lanosterol treatment was compared, obtaining a solid trend towards reduction of cataract severity, as evidenced by an increase in lens clarity. The information shown in this patent does not differ from that reported in other references on the limitations found for the use of lanosterol in the treatment of cataracts, particularly as reported by Shanmugam *et al.,* using the same concentration of lanosterol.

Patent CN106344587A describes a preparation of a lanosterol compound for eyes, particularly a pharmaceutical composition comprising 5-250 mM lanosterol compounds and a method of preparing the pharmaceutical composition, as well as the application of the pharmaceutical composition regarding prevention and treatment of ophthalmic diseases. The described composition in the form of an eye drop containing 25 mM lanosterol (11.4 mg/mL), administered to *Canis familiaris* L., was applied 3 times daily: 50 µL of 25 mM lanosterol in the morning, afternoon and evening at intervals of at least 5 hours between each administration, for 12 successive weeks. Although no express data are shown, it is proposed that this composition may completely cure traumatic cataracts in 2 weeks, although the aforementioned subsequent experimental evidence does not support its use, particularly in advanced cataracts of higher grades.

Chemical modifications to lanosterol have also been proposed to enhance its effects. Patent application WO2019097434A1 relates to a composition of lanosterol and 25-hydroxycholesterol derivatives, including their pharmaceutically acceptable salts, used in combination with oxidative protectants, free radical scavengers, modulators of protein carbonylation, lipid peroxidation, redox enzyme enhancement or antioxidants. The document proposes an inhibitory concentration of lanosterol derivatives between 0.010% w/v to about 5% w/v. A patient diagnosed with cataract and showing clouding of the lens is photographically imaged prior to treatment. Although the paper reports "dramatic improvement in lens clouding after 3 weeks with almost complete absence of clouding after 6 weeks," a measurement of grade reduction is not reported in terms of cataract developmental stage. Therefore, it is unclear whether the modifications made may serve in cases of cataracts at advanced developmental stages.

Patent application US2020016176A1 describes an aqueous ophthalmic composition for the treatment of ocular diseases, injuries or damage, comprising a steroid such as lanosterol in combination with others. However, it highlights the importance of the excipient in the final therapeutic effect, using 2-hydroxypropyl-β-cyclodextrin (CD) and hydroxypropyl methylcellulose (HPMC). They accurately performed studies on the treatment of cataractous lens in dogs of 3 different breeds using as a procedure the injection of nanoparticles loaded with steroid formulation (100 µg). These included lanosterol and another cholesterol-derived steroids, with a volume equivalent to 50 µL (1 drop), 3 times a day at around 7 A.M., 1 P.M. and 4 P.M., for 3 weeks. Experimental data show a maximum improvement of 20% in this experiment in the dogs used.

Other bioactive compounds that have been tried for the treatment of cataracts are polyphenolic compounds of natural origin, as is particularly the curcumin case.

It has been reported that curcumin may inhibit sodium selenite-induced oxidative stress, as is the case Manikandan *et al.* (Manikandan et al. Effect of Curcumin on Selenite-Induced Cataractgenesis in Wistar Rat Pups. Current Eye Research. 2010; 35:122-129). They studied the antioxidant potential of curcumin on sodium selenite-induced (15 µM/kg body weight) cataract in rat pups. This group of researchers concluded that treatment with curcumin (75 mg/kg body weight as a single dose) led to a significant reduction in the levels of lipid peroxidation, enzymatic antioxidants and non-enzymatic antioxidants, which may support that consumption of free curcumin in food could help prevent the onset of senile cataracts. In another work, Manikandan *et al.* (Manikandan et al. Effect of Curcumin on the Modulation of αA- and αB-crystallin and Heat Shock Protein 70 in Selenium-Induced Cataractgenesis in Wistar Rat Pups. Molecular Vision. 2011; 17:388-394.) studied the expression of αA- and αB-crystallin and heat shock protein 70 (Hsp 70) during curcumin treatment of sodium selenite-induced cataractogenesis in Wistar rat pups. They similarly concluded that curcumin suppressed αA- and αB-crystallin expression induced by selenite and Hsp 70. Therefore, curcumin may suppress and/or prevent cataract formation in rat pups.

Regarding the effectiveness of curcumin in prevention and treatment, Yogaraj *et al.* (Yogaraj et al. Quaternary Ammonium Dendrimeric Poly (Amidoamine) Encapsulated Nanocurcumin Effectively Prevents Cataracts in Rat Pups by Regulating Pro-Inflammatory Gene Expression. Journal of Drug Delivery Science and Technology. 2020; 58:1773-2247) developed and characterized a nanocurcumin formulation employing a functionalized polyamidoamine dendrimer (PAMAM) (third generation) with encapsulated curcumin. They tested their formulation in the same model as Manikandan's group, in Wistar rat pups by expressing proinflammatory genes using *in vitro* human lens epithelial cell (HLE-B3) systems. Their results show that nanocurcumin proved to be as effective as free curcumin in reducing cell death, RNA degradation and iNOS (inducible nitric oxide synthase) level gene expression induced by sodium selenite. Therefore, they conclude that the encapsulated curcumin formulation using QPAMAM compensates for the main limitations of its free counterpart with higher solubility, sustained release and higher bioavailability by preventing oxidant-mediated cataract development.

Publication CN106511269A also describes an ophthalmic nanosuspension preparation, as well as a preparation method. Particularly, the nanosuspension ophthalmic preparation is prepared by combining an ophthalmic preparation pharmaceutical adjuvant with curcumin. The ophthalmically acceptable gel and nanosuspension preparation of curcumin was tested in the same rat model with selenite-induced cataract. Administration on a 3 times/8 days regime showed a beneficial effect of curcumin ophthalmic nanosuspension. There is evidence of a better bioavailability, with a larger surface area, to improve the degree of drug absorption, thus extending time of action. However, it is not demonstrated in *vivo* that reversion is achieved, but rather the enzymatic activity is characterized ex *vivo.*

Patent EP2346520B1 relates to an aqueous ophthalmic composition comprising natural curcuminoids, such as curcumin, bisdemethoxycurcumin, demethoxycurcumin and synthetically derived bis-o-demethyl curcumin and/or other demethylated curcuminoids, isolated or in combination, with suitable surfactants and co-solvents as penetration enhancers, along with other ophthalmic excipients, useful for the treatment of ocular diseases or disorders. Similar to the other curcumin-related papers, it is concluded that instillation of the 98% curcumin ophthalmic formulation into the eye of Wistar rat pups effectively reduced the effect of selenite-induced cataract, having a prevention effect.

However, experimental evidence regarding polyphenolic compounds of natural origin, such as curcumin, has not shown that they can reverse the formation of cataracts with high developmental degrees, but only prevent their formation. Similarly, the development of compositions based on terpenoid compounds such as lanosterol has achieved a minimal reduction in cataracts, by using repetitive and very aggressive treatments, with high doses. Efforts to develop compositions based on these types of bioactive compounds have not achieved significant reduction or elimination of cataracts.

As a result of the above, it has been sought to solve the issues of the ophthalmic compositions for the treatment of visual disorders currently used, by developing a composition that, in addition to allowing the reversion of cataracts in advanced developmental stages, requires low doses and a reduced number of applications to be effective.

### OBJECT OF THE INVENTION

Taking into account the defects of the prior art, one of the objects of this invention is to provide an ophthalmic composition for the treatment of visual disorders requiring low doses of bioactive compounds to reverse and/or prevent alterations in the regular structure of the eye's lens, mainly those conditions modifying or altering the proteins that form it.

Another object of this invention is to provide an ophthalmic composition for the treatment of visual disorders which requires a reduced number of applications to be effective in the treatment of this kind of disorders.

These and other subjects are achieved by an ophthalmic composition for the treatment of visual disorders in accordance with this invention.

### SUMMARY OF THE INVENTION

In order to achieve the objectives of this invention, an ophthalmic composition for the treatment of visual disorders has been invented. This is characterized in that it comprises a polyphenolic compound in combination with a terpenoid, in therapeutically effective amounts, allowing to revert and/or prevent alterations in the regular structure of the lens, mainly those conditions that modify or alter the proteins that form it.

Other aspects of the invention consider the manufacture method for the ophthalmic composition of this invention, as well as its use in the treatment of ophthalmic diseases caused by the alteration or aggregation of proteins.

### BRIEF DESCRIPTION OF THE FIGURES

The novel aspects considered to be characteristic of this invention will be set forth in detail in the attached claims. However, some embodiments, characteristics, as well as some objects and advantages thereof, will be better understood in the detailed description, when read in relation to the attached drawings, in which:
Figure 1 is a set of photographic views of the different stages of lens opacification in experimental animals in order to illustrate the different cataract developmental degrees.
Figure 2 is a set of photographic views of healthy-control and diseased-control animals' lens with grade-4 cataract development.

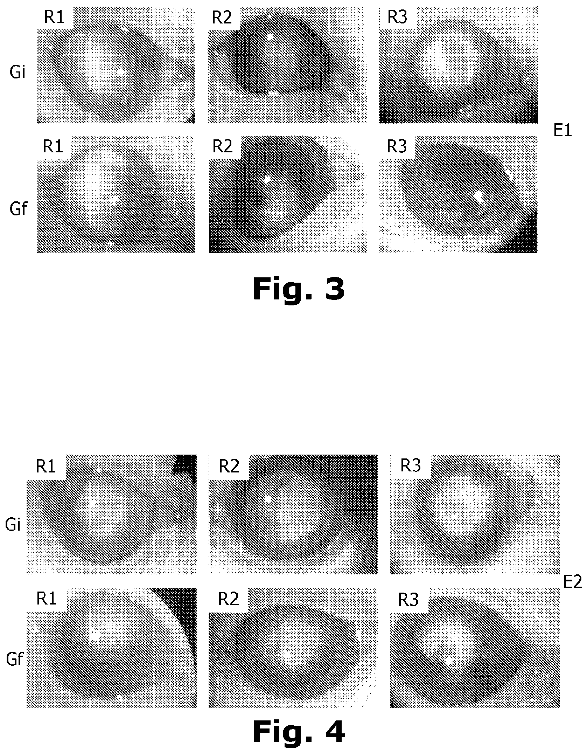

Figure 3 is a set of photographic views of animals' lens before and after treatment with a curcumin nanoemulsion intravitreally applied.
Figure 4 is a set of photographic views of animals' lens before and after treatment with a lanosterol nanoemulsion intravitreally applied.

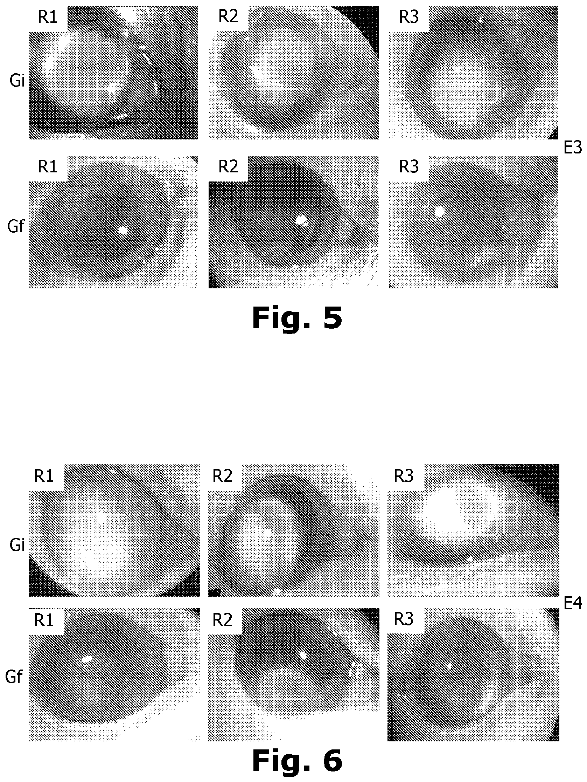

Figure 5 is a set of photographic views of animals' lens before and after treatment with a curcumin nanoemulsion containing lanosterol in a 1:1 ratio, intravitreally applied.
Figure 6 is a set of photographic views of animals' lens before and after treatment with a curcumin nanoemulsion containing lanosterol in a 1:3 ratio, intravitreally applied.

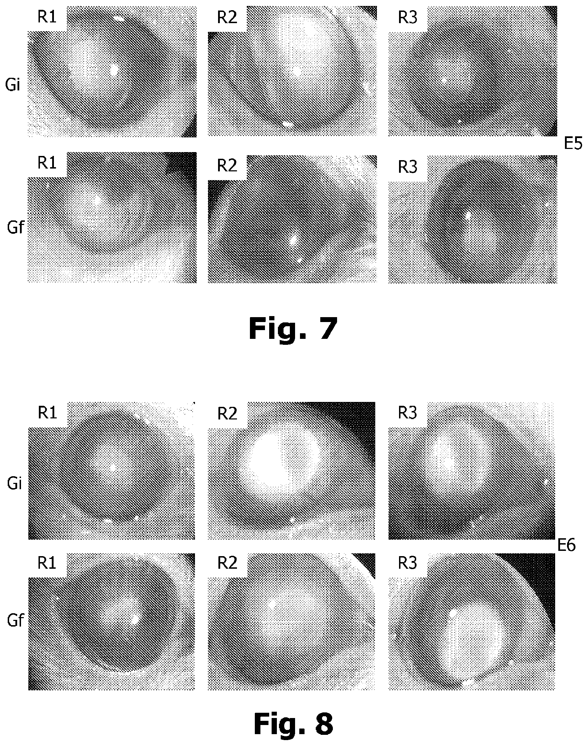

Figure 7 is a set of photographic views of animals' lens before and after treatment with free curcumin intravitreally applied.
Figure 8 is a set of photographic views of animals' lens before and after treatment with free lanosterol intravitreally applied.

### DETAILED DESCRIPTION OF THE INVENTION

It has been found that it is possible to reverse and/or prevent alterations in the regular structure of the lens, mainly those conditions modifying or altering the proteins that form the lens, in several known visual disorders, by a small number of applications, and to effectively treat those visual disorders by means of an ophthalmic composition comprising a polyphenolic compound in combination with a terpenoid, or of their respective pharmaceutically acceptable salts or crystalline forms, in therapeutically effective amounts.

The new combination of polyphenolic compounds with terpenoids exhibits a synergistic effect that allows reversing and/or preventing alterations in the lens' structure at lower doses and more effectively than those reported in the state of the art for both compounds individually, through a smaller number of applications and in less time.

According to the principles of this invention, ophthalmic compositions may comprise any ratio between polyphenolic compounds and terpenoids. In a preferred embodiment, the ophthalmic compositions of this invention comprise the polyphenolic compound in a ratio of 1:10 to 10:1 regarding the terpenoid, preferably in a 1:3 to 3:1 ratio of the polyphenolic compound regarding the terpenoid, and more preferably both compounds being present in the ophthalmic composition in a 1:1 ratio.

In a preferred embodiment of this invention, the polyphenolic compound is a natural curcuminoid, preferably selected from curcumin; bisdemethoxycurcumin; demethoxycurcumin and synthetically derived bis-o-demethyl curcumin, and/or other demethylated curcuminoids; the stable isomers of the foregoing; or their pharmaceutically acceptable salts or crystalline forms, curcumin of natural origin being particularly preferred due to its antioxidant and anti-inflammatory activities. These compounds have been sufficiently described in the state of the art so that a skilled person may obtain them by using those techniques previously described in the background of the invention.

The terpenoid is selected from lanosterol and its steroid derivatives, dihydrolanosterol; 4,4-dimethylcholesta-8(9),14,24-trien-3β-ol; 4,4-dimethylcholesta-8,24-dien-3β-ol; 4,4-dimethylcholesta-8-en-3β-ol; 4,4-dimethylcholesta-8(9),14-dien-3β-ol; 14-desmethyl lanosterol; latosterol; Δ7.24-cholestadienol; cholesterol; cholesta-7-enol; cholesterol ester; 7-dehydrocholesterol; desmosterol; 7-dehydrodesmosterol; zymosterol; 27-hydroxycholesterol; cholesta-7,24-dien-3-β-ol; cholesta-8(9)-en-3-β-ol; 5α-cholestan-3β-6-one; 5-cholesten-3β,25-diol; 5-cholesten-3β,25-OSO3H (5-cholesten-3β, 25-sulfate); 5-cholesten-3β-OSO3H,25-ol (5-cholesten-3β-sulfate,25-ol); 5-cholesten-3β,25-diol; disulfate and/or esters thereof; stable isomers of the foregoing; or pharmaceutically acceptable salts or crystalline forms thereof, lanosterol and/or its derivatives being particularly preferred. Similarly, these compounds have been described in the state of the art so that their obtaining is evident for a skilled person by referring to the known techniques in the background of the invention.

In a particularly preferred embodiment, the composition comprises curcumin as a polyphenolic compound, preferably obtained from the rhizome of the herbaceous perennial plant *Curcuma longa* using the techniques described in the state of the art.

In another particularly preferred embodiment, the composition comprises lanosterol as a tetracyclic terpenoid of an amphipathic nature, synthesized from squalene or waxes such as lanolin, also using the techniques described in the state of the art.

Regarding other ophthalmically acceptable components to prepare the ophthalmic composition of this invention, it is preferred that these be properly selected for application of the composition by topical, subconjunctival, retrobulbar, periocular, subretinal, suprachoroidal, intracameral, intravitreal, or intraocular routes, including but not limited to pharmaceutical forms selected from ophthalmic solution, ophthalmic ointment, ophthalmic wash, intraocular infusion solution, anterior chamber wash, internal medicine, injection, as part of an ocular implant, or as a preservative for the extracted cornea. In order to formulate the compositions of this invention, it is further possible to use ophthalmically acceptable components and excipients including, but not limited to water, buffer or sodium chloride solutions, surfactants or cyclodextrins, emulsions, liposomes, ophthalmically acceptable gels or suspensions, or combinations thereof. A particularly preferred excipient consists of ophthalmic nanoemulsions.

The compositions of this invention are effective for reversing and/or preventing alterations of lens structure, primarily alteration of lens proteins, preferably by at least a single application of the composition. For this reason, they are useful for the treatment of visual disorders, including cataracts, regardless of their cause, or other diseases related to changes in the structure of the lens, such as presbyopia, lens sclerosis, retinal degeneration, Refsum disease, Smith-Lemli-Opitz syndrome (SLOS), drusen, Schnyder corneal dystrophy (SCD), abetalipoproteinemia (ABL), familial hypobetalipoproteinemia (FHBL), age, macular degeneration, and diabetic retinopathy. When the disease is cataracts, the composition of this invention is capable of virtually completely reversing the disease.

Although the techniques to prepare the compositions of this invention are known in the state of the art, it may illustratively be said, for the inclusion of the polyphenolic compounds and terpenoids in an ophthalmic nanoemulsion, that it is possible to use any known process described in the state of the art, such as the one reported by Agame-Lagunes *et al.* (Agame-Lagunes et al. Curcumin Nanoemulsions Stabilized with Modified Phosphatidylcholine on Skin Carcinogenesis Protocol. Current Drug Metabolism. 2020, 21, (3):226-234), generally consisting of mixing an oily phase comprising said compounds and an aqueous phase, in order to obtain nanoemulsions through methods known in the state of the art, either high- or low-energy, high-energy ones being preferred. Those methods make it possible to obtain a particle size on a nanometer scale ranging from 5 to 999 nm, preferably between a range from 20 to 300 nm from one phase, preferably the oily phase.

In a specific embodiment, for the nanoemulsion formation, a mixture is provided with a ratio between 80:20 and 95:5 of the aqueous phase regarding the oily phase, being particularly preferred mixtures with at least 95% content of the aqueous phase.

In order to prepare the nanoemulsions of the compounds for this invention, an ophthalmically acceptable oil, surfactant and organic solvent are preferably included in the oily phase. An oil is preferred, more preferably a triacylglycerides oil, more preferably with a glycerol derivative and three fatty acids, the mixture of medium chain triacylglycerides being preferred. The surfactant is also preferred to be amphiphilic of synthetic or natural origin, preferably natural, such as phosphatidylcholine, mono- and diacylglycerides, more preferably phosphatidylcholine. The ophthalmically acceptable organic solvent is preferred to be an alcohol, more preferably from 1 to 3 carbon atoms, ethanol being particularly preferred.

As regards the aqueous phase, a preferred embodiment comprises deionized water and glycerol as a stabilizer, in order to form an ophthalmically acceptable excipient. In a preferred embodiment of this invention, the aqueous phase comprises from 5% to 60% glycerol, a range from 10% to 45% glycerol being preferred, and amounts from 15% to 25% glycerol still more preferred.

The ophthalmic composition obtained in accordance with the principles of this invention is prepared for its use in the treatment and/or prevention of visual disorders caused by changes in the structure of the eye's lens, particularly related to protein alteration, in any pharmaceutical form useful for application to the eye. This is used to reverse said visual disorder almost completely, and it is adaptable to be preferably used in at least one application, in order to achieve a reversal in a maximum of 7 days following treatment.

This invention will be better understood with the following examples. They are presented only for illustrative purposes to allow a thorough understanding of the preferred embodiments of this invention and to guide to their realization. This does not imply there are no other embodiments, not illustrated, which may be implemented based on the aforementioned description.

### EXAMPLES

### A. Preparation of ophthalmic compositions for the state of the art and this invention.

In order to illustrate the novel effects of the compositions for this invention, 6 examples of compositions shown in Table 1 were made with a base of 10 mL, as sterile nanoemulsions, for its intravitreal application, using curcumin as polyphenol, and lanosterol as terpenoid, as they are the most studied ones in the state of the art:

**Table 1 - Ophthalmic compositions assessed**

| **No.** | | **Curcumin (C) (mg/mL)** | **Lanosterol (L) (mg/mL)** | **Ratio C:L** | **Average particle size (nm)** |
|---|---|---|---|---|---|
| **E1** | **CN** | 2 | 0 | N/A | 150.7 ± 9.10 |
| **E2** | **LN** | 0 | 2 | N/A | 100.7 ± 1.10 |
| **E3** | **CLN1:1** | 1 | 1 | 1:1 | 108.3 ± 1.2 |
| **E4** | **CLN1:3** | 0.5 | 1.5 | 0.5:1.5 | 119.3 ± 2.4 |
| **E5** | **C** | 2 | 0 | N/A | N/A |
| **E6** | **L** | 0 | 2 | N/A | N/A |

In order to prepare the nanoemulsions to be tested, an oily phase (to be dispersed) was obtained with curcumin and/or lanosterol according to the Table, along with 10% phosphatidylcholine, 3 mL ethanol and 5% of a medium chain triacylglycerides mixture. Once the mixture of the compounds for this invention has been made, prior to homogenization, the oily phase was subjected to water bath to remove the excess of organic solvent.

The aqueous phase (dispersant) consisted of 60% water and 25% glycerol. The inclusion of both phases was performed manually for further processing in the ULTRA-TURRAX digital T25 rotor-stator homogenizer (IKA Works, Inc. Staufen, Germany) to create a coarse emulsion, which was then subjected to ultrasonication to decrease the particle size using a Branson Digital Sonifier S-450D (Branson Ultrasonic Corp., Danbury, CT) to obtain the pertinent nanoemulsion.

Compositions were sterilized using an UV light lamp. For sterilization, the systems were poured into sterile culture plates with a capacity of 10 mL and then left uncovered under an UV light lamp for 40 minutes. The systems were then collected with a sterile syringe in glass bottles sealed with parafilm until their use in the animal model. All procedures were performed under sterile conditions.

In addition, compositions E5 and E6 were prepared, with free curcumin (C) and free lanosterol (L), respectively, which were prepared by dissolving only the compounds in the dispersed phase used. In this case, the mixture of medium chain triacylglycerides was performed with a ratio in accordance with the Table, having a base of 10 mL.

### B. Cataract induction in Wistar rats.

In order to perform the assessment of cataract reversion *in vivo,* 7-week-old albino Wistar rats weighing between 120-140 g, randomly assigned in groups (n=3), were kept in a confined area, in a controlled environment with a temperature of 23 ± 2 °C, as well as relative humidity between 40 and 70%, with cycles of 12 hours of light and 12 hours of darkness, and were provided with a commercial diet and water *ad libitum.* The animals were cared for in accordance with the specifications of Mexican Official Standard NOM-062-ZOO-1999.

Thirty rats were administered sodium selenite intravitreally to develop cataracts. After treatment, the experimental animals developed the disease, but of these animals, only 26.66% developed cataracts in both eyes. The rest developed the disease in only one eye, with prevalence in the left eye.

For intravitreal treatment, rats were anesthetized by intraperitoneal injection of Zoletil 50 (40 mg/kg body weight), in addition to local anesthetic (tetracaine), as well as eye lubricant (hypromellose) and antibiotic (ciprofloxacin) to avoid infection.

To perform the intravitreal injection, a stereoscope was handled to have greater accuracy during the procedure. 1 mL insulin syringes with 27S gauge needles were used to make a puncture hole at a 45° angle through the sclera into the vitreous body in order to release intraocular pressure and remove some vitreous humor. A Hamilton syringe with a 26S gauge removable needle was inserted into that same hole with selenite solution, which was injected into the chamber avoiding touching the lens. The needle was then carefully removed, and a drop of antibiotic was placed in each eye.

The opacity assessment following intravitreal sodium selenite administration was performed weekly for 4 weeks by slit-lamp microscopy, while the classification of lens opacity was performed as shown in Table 2 below:

**Table 2 - Grades of lens opacity**

| | |
|---|---|
| Grade 0 (G0) | Lack of opacity |
| Grade 1 (G1) | Slight degree of opacity |
| Grade 2 (G2) | Presence of diffuse opacity involving lens areas |
| Grade 3 (G3) | Presence of dense opacity involving lens areas |
| Grade 4 (G4) | Presence of extensive and dense opacity involving the entire lens |

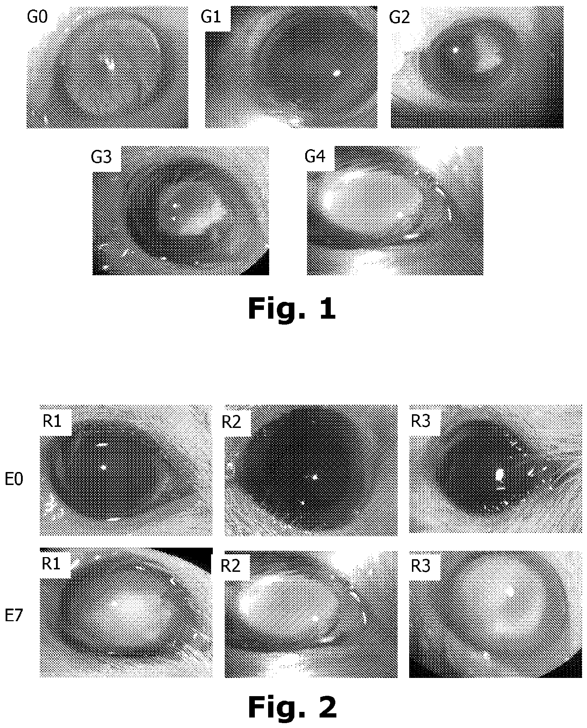

The degrees of cataract development are shown in Figure 1, in which photographic views of lens opacification in experimental animals may be evidenced. Figure 1 shows the grade-zero lens (G0), grade-one lens (G1), grade-two lens (G2), grade-three lens (G3), and finally grade-four lens (G4).

### C. Application of ophthalmic compositions to rats with cataracts

Eight groups of three albino Wistar rats were formed. A healthy control negative control group (E0) was not induced with sodium selenite and did not develop cataracts, while another positive control or diseased control group (E7) was induced with cataracts as described above, but did not receive treatment with curcumin or lanosterol or their mixtures of this invention. The rest of the groups were respectively treated with compositions E1 to E6 intravitreally, using the same technique as that used for the application of sodium selenite, with a volume of 5 µL of each ophthalmic composition, in two applications (except the control groups) one week apart, obtaining the results in Table 3 for each rat (R1 to R3).

**Table 3 - Results of treatment with ophthalmic compositions and control groups E0 to E7**

| **No.** | **Treatment** | **Initial opacity grade (Gi)** | | | **Final opacity grade (Gf)** | | | **Average final opacity** | **Illustrative figure** |
|---|---|---|---|---|---|---|---|---|---|
| | | R1 | R2 | R3 | R1 | R2 | R3 | | |
| **E0** | **None** | G0 | G0 | G0 | G0 | G0 | G0 | 0 | Fig. 2 |
| **E1** | **CN** | G4 | G4 | G4 | G4 | G2 | G2 | 2.66 | Fig. 3 |
| **E2** | **LN** | G4 | G4 | G4 | G2 | G3 | G3 | 2.66 | Fig. 4 |
| **E3** | **CLN1:1** | G4 | G4 | G4 | G1 | G1 | G1 | 1 | Fig. 5 |
| **E4** | **CLN1:3** | G4 | G4 | G4 | G1 | G2 | G1 | 1.33 | Fig. 6 |
| **E5** | **C** | G4 | G4 | G4 | G3 | G1 | G3 | 2.33 | Fig. 7 |
| **E6** | **L** | G4 | G4 | G4 | G3 | G4 | G4 | 3.66 | Fig. 8 |
| **E7** | **None** | G4 | G4 | G4 | G4 | G4 | G4 | 4 | Fig. 2 |

The degrees of cataract development and its reduction may be evidenced in the Figures shown in Table 3, whereby the synergistic effect obtained from the compositions of this invention (E3 and E4), which managed to reverse almost all the opacity, may be observed. This even occurred with composition E4, where a significantly lower amount of curcumin with lanosterol achieved a more than double of reversion, even considering that a skilled person would not be motivated to use more lanosterol than curcumin, based on the results of the state of the art and the results obtained for examples E5 and E6, which showed better reversion with curcumin than with free lanosterol.

All of the above is evidenced when analyzing the final opacity after treatment, which in the groups of the compositions for this invention were almost 1. This is highly statistically significant as checked by Duncan's statistical analysis illustrated in Table 4, where groups E4 and E3 are in subset 2, having similar results. In Table 4, the final opacity means for the groups in the homogeneous subsets are displayed, and the harmonic mean sample size = 3,000 is used.

In this regard, according to Duncan's statistical analysis, the best treatment corresponds to E3, a group that is found in subset 1 along with E0, stating that E3 composition was able to reverse cataracts in a remarkable way by reporting that there is no statistically significant difference with E0 (healthy control).

**Table 4 - Duncan's statistical analysis of the average final opacity of groups E0 to E7**

| **No.** | **N** | **Significance level = 0.05** | | | | | |
|---|---|---|---|---|---|---|---|
| | | **1** | **2** | **3** | **4** | **5** | **6** |
| E0 | 3 | 0.0000 | | | | | |
| E3 | 3 | 1.0000 | 1.0000 | | | | |
| E4 | 3 | | 1.3333 | 1.3333 | | | |
| E5 | 3 | | | 2.3333 | 2.3333 | | |
| E1 | 3 | | | | 2.6667 | 2.6667 | |
| E2 | 3 | | | | 2.6667 | 2.6667 | |
| E6 | 3 | | | | | 3.6667 | 3.6667 |
| E7 | 3 | | | | | | 4.0000 |
| Sig. | | 0.089 | 0.555 | 0.089 | 0.576 | 0.105 | 0.555 |

It is also remarkable that the difference in the final opacity means between the minimum and maximum of statistical group 3 is one degree (1.3333 to 2.3333), while between the minimum and maximum of group 2 is 0.3333 (1.0000 to 1.3333). This shows that the treatments with the compositions for this invention are highly effective in the treatment of visual disorders.

Thus, it has been demonstrated that under the principles of this invention, novel effects superior to those obtained by the compounds separately are achieved. In addition, it will be evident to a skilled person that it is possible to make combinations of other polyphenolic compounds with other terpenoids, which have already demonstrated in the state of the art a much less significant cataract reversal effect on their own, equivalent to examples E1, E2, E5 or E6, to obtain a synergistic effect equal to that obtained with compositions E3 and E4 illustrated in the aforementioned examples.

In accordance with the state of the art, it will also be evident to a skilled person that the compositions of this invention may be formulated in different pharmaceutical forms for ophthalmic use. This is because the state of the art itself has illustrated the effects of the separate compounds in several ophthalmic compositions, such as topical, subconjunctival, retrobulbar, periocular, subretinal, suprachoroidal, suprachoroidal, intracameral, intravitreal or intraocular route.

In accordance with the foregoing, it will be evidenced that the ophthalmic compositions for the treatment of visual disorders for this invention have been prepared to achieve the reversal and/or prevention of such disorders caused by changes in the structure of the lens, mainly by the alteration of lens proteins. It will also be evident to anyone skilled in the art that the embodiments of such compositions, as described above and illustrated in the attached Figures, are only illustrative but not limiting of this invention, since numerous changes of consideration are possible in their details without departing from the scope of invention.

Therefore, this invention should not be considered as restricted except as required by the state of the art and by the scope of the attached claims.

## Claims

1. An ophthalmic composition **characterized in that** it comprises a polyphenolic compound and a terpenoid, or their respective pharmaceutically acceptable salts or crystalline forms, in therapeutically effective amounts.

2. The ophthalmic composition according to claim 1, further **characterized in that** the polyphenolic compound is selected from natural curcuminoids or derivatives thereof.

3. The ophthalmic composition according to claim 2, further **characterized in that** the polyphenolic compound is selected from curcumin, bisdemethoxycurcumin, demethoxycurcumin and synthetically derived bis-o-demethyl curcumin and/or other demethylated curcuminoids, or stable isomers of the foregoing.

4. The ophthalmic composition according to claim 3, further **characterized in that** the polyphenolic compound is natural curcumin.

5. The ophthalmic composition according to claim 1, further **characterized in that** the terpenoid is selected from lanosterol and its steroid derivatives.

6. The ophthalmic composition according to claim 5, further **characterized in that** the terpenoid is selected from lanosterol; dihydrolanosterol; 4,4-dimethylcholesta-8(9),14,24-trien-3β-ol; 4,4-dimethylcholesta-8,24-dien-3β-ol; 4,4-dimethylcholesta-8-en-3β-ol; 4,4-dimethylcholesta-8(9),14-dien-3β-ol; 14-desmethyl lanosterol; latosterol; Δ7.24-cholestadienol; cholesterol; cholesta-7-enol; cholesterol ester; 7-dehydrocholesterol; desmosterol; 7-dehydrodesmosterol; zymosterol; 27-hydroxycholesterol; cholesta-7,24-dien-3-β-ol; cholesta-8(9)-en-3-β-ol; 5α-cholestan-3β-6-one; 5-cholesten-3β,25-diol; 5-cholesten-3β,25-OSO3H (5-cholesten-3β, 25-sulfate); 5-cholesten-3β-OSO3H,25-ol (5-cholesten-3β-sulfate,25-ol); 5-cholesten-3β,25-diol; disulfate and/or esters thereof; or stable isomers of the foregoing.

7. The ophthalmic composition according to claim 6, further **characterized in that** the terpenoid is lanosterol.

8. The ophthalmic composition according to claim 4, further **characterized in that** the curcumin is derived from the rhizome of the herbaceous perennial plant *Curcuma longa.*

9. The ophthalmic composition according to claim 7, further **characterized in that** the terpenoid is lanosterol as a tetracyclic terpenoid of amphipathic nature, synthesized from squalene or waxes such as lanolin.

10. The ophthalmic composition according to claim 1, further **characterized in that** it comprises an ophthalmically acceptable excipient for topical, subconjunctival, retrobulbar, periocular, subretinal, suprachoroidal, intracameral, intravitreal or intraocular application.

11. The ophthalmic composition according to claim 10, further **characterized in that** its pharmaceutical form is selected from ophthalmic solution, ophthalmic ointment, ophthalmic wash, intraocular infusion solution, anterior chamber wash, internal medicine, injection, as part of an ocular implant or as a preservative for the extracted cornea.

12. The ophthalmic composition according to claim 10, further **characterized in that** the excipient is selected from water, buffer or sodium chloride solutions, surfactants or cyclodextrins, emulsions, liposomes, ophthalmically acceptable gels or suspensions, or combinations thereof.

13. The ophthalmic composition according to claim 10, further **characterized in that** the ophthalmically acceptable excipient is an ophthalmic nanoemulsion.

14. The ophthalmic composition according to claim 13, further **characterized in that** it comprises an oily phase with average particle size between 5 and 999 nm nanoemulsified in an aqueous phase.

15. The ophthalmic composition according to claim 14, further **characterized in that** it comprises an oily phase with average particle size between 20 and 300 nm.

16. The ophthalmic composition according to claim 14, further **characterized in that** the oily phase comprises an oil, a surfactant and an ophthalmically acceptable organic solvent.

17. The ophthalmic composition according to claim 16, further **characterized in that** the oil of the oily phase is a triacylglycerides oil.

18. The ophthalmic composition according to claim 17, further **characterized in that** the oil in the oily phase is a glycerol derivative and three fatty acids.

19. The ophthalmic composition according to claim 17, further **characterized in that** the oil in the oily phase is a medium chain triacylglycerides mixture.

20. The ophthalmic composition according to claim 16, further **characterized in that** the surfactant is amphiphilic of synthetic or natural origin.

21. The ophthalmic composition according to claim 20, further **characterized in that** the surfactant is selected from phosphatidylcholine and mono- and diacylglycerides.

22. The ophthalmic composition according to claim 16, further **characterized in that** the ophthalmically acceptable organic solvent is an alcohol.

23. The ophthalmic composition according to claim 22, further **characterized in that** the alcohol has from 1 to 3 carbon atoms.

24. The ophthalmic composition according to claim 23, further **characterized in that** the alcohol is ethanol.

25. The ophthalmic composition according to claim 14, further **characterized in that** the aqueous phase comprises deionized water and glycerol as a stabilizer.

26. An ophthalmic composition according to any one of claims 1 to 25, to be used in the treatment and/or prevention of an ophthalmic disease caused by alterations in the regular lens structure, mainly those conditions modifying or altering the proteins that form the lens.

27. The ophthalmic composition according to claim 26, further **characterized in that** the ophthalmic disease is selected from cataract, presbyopia, lens sclerosis, retinal degeneration, Refsum disease, Smith-Lemli-Opitz syndrome (SLOS), drusen, Schnyder corneal dystrophy (SCD), abetalipoproteinemia (ABL), familial hypobetalipoproteinemia (FHBL), age, macular degeneration, and diabetic retinopathy.

28. The ophthalmic composition according to claim 26, further **characterized in that** the ophthalmic disease is treated or prevented by at least a single application of the composition.

29. The ophthalmic composition according to claim 26, further **characterized in that**, when the disease is cataracts, the composition of this invention is capable of reversing to the minimum degree the opacity caused by the disease.

30. The ophthalmic composition according to claim 26, further **characterized in that** the ophthalmic disease is treated or prevented by up to two applications of the composition intravitreally.

31. The use of a polyphenolic compound in combination with a terpenoid, or its salts or pharmaceutically acceptable crystalline forms, to manufacture a medicine for treating and/or preventing an ophthalmic disease caused by alterations in the regular lens structure, including those conditions modifying or altering the proteins that form the lens.

32. The use according to claim 31, wherein the polyphenolic compound is selected from natural curcuminoids or derivatives thereof.

33. The use according to claim 32, wherein the polyphenolic compound is selected from curcumin, bisdemethoxycurcumin, demethoxycurcumin and synthetically derived bis-o-demethyl curcumin and/or other demethylated curcuminoids, or stable isomers of the foregoing.

34. The use according to claim 32, wherein the polyphenolic compound is natural curcumin.

35. The use according to claim 31, wherein the terpenoid is selected from lanosterol and its steroid derivatives.

36. The use according to claim 35, wherein the terpenoid is selected from lanosterol; dihydrolanosterol; 4,4-dimethylcholesta-8(9),14,24-trien-3β-ol; 4,4-dimethylcholesta-8,24-dien-3β-ol; 4,4-dimethylcholesta-8-en-3β-ol; 4,4-dimethylcholesta-8(9),14-dien-3β-ol; 14-desmethyl lanosterol; latosterol; Δ7.24-cholestadienol; cholesterol; cholesta-7-enol; cholesterol ester; 7-dehydrocholesterol; desmosterol; 7-dehydrodesmosterol; zymosterol; 27-hydroxycholesterol; cholesta-7,24-dien-3-β-ol; cholesta-8(9)-en-3-β-ol; 5α-cholestan-3β-6-one; 5-cholesten-3β,25-diol; 5-cholesten-3β,25-OSO3H (5-cholesten-3β, 25-sulfate); 5-cholesten-3β-OSO3H,25-ol (5-cholesten-3β-sulfate,25-ol); 5-cholesten-3β,25-diol; disulfate and/or esters thereof; or stable isomers of the foregoing.

37. The use according to claim 35, wherein the terpenoid is lanosterol.

38. The use according to claim 34, wherein the curcumin is derived from the rhizome of the herbaceous perennial plant *Curcuma longa.*

39. The use according to claim 37, wherein the terpenoid is lanosterol as a tetracyclic terpenoid of amphipathic nature, synthesized from squalene or waxes such as lanolin.

40. The use according to claim 31, wherein the ophthalmic disease is selected from cataract, presbyopia, lens sclerosis, retinal degeneration, Refsum disease, Smith-Lemli-Opitz syndrome (SLOS), drusen, Schnyder corneal dystrophy (SCD), abetalipoproteinemia (ABL), familial hypobetalipoproteinemia (FHBL), age, macular degeneration, and diabetic retinopathy.
